Europäisches Patentamt

European Patent Office

Office européen des brevets

(19)

(11) EP 0 970 118 B1

(12) FASCICULE DE BREVET EUROPEEN

(45) Date de publication et mention
de la délivrance du brevet:
**18.08.2004 Bulletin 2004/34**

(51) Int Cl.⁷: **C07K 14/47**, A61K 31/00,
A61K 38/00

(21) Numéro de dépôt: **98902026.8**

(22) Date de dépôt: **06.01.1998**

(86) Numéro de dépôt international:
**PCT/FR1998/000009**

(87) Numéro de publication internationale:
**WO 1998/030588 (16.07.1998 Gazette 1998/28)**

(54) **INHIBITEURS SPECIFIQUES DE LA LIPASE PANCREATIQUE ET LEURS APPLICATIONS**

SPEZIFISCHE INHIBITOREN DER PANKREATISCHEN LIPASE UND DEREN ANWENDUNGEN

SPECIFIC PANCREATIC LIPASE INHIBITORS AND THEIR APPLICATIONS

(84) Etats contractants désignés:
**AT BE CH DE DK ES FI FR GB GR IE IT LI LU MC NL PT SE**

(30) Priorité: **07.01.1997 FR 9700071**

(43) Date de publication de la demande:
**12.01.2000 Bulletin 2000/02**

(73) Titulaire: **LIPOGEL**
**13190 Allauch (FR)**

(72) Inventeur: **CHAPUS, Catherine**
**F-13006 Marseille (FR)**

(74) Mandataire: **Goulard, Sophie et al**
**Cabinet ORES,**
**36, rue de St Pétersbourg**
**75008 Paris Cedex (FR)**

(56) Documents cités:
**EP-A- 0 399 379**

- **BOUSSET-RISSO M. ET AL.: "Limited proteolysis of porcine pancreatic lipase. Lability of the Phe 335 - Ala 336 bond towards chimotrypsin" FEBS LETTERS, vol. 182, no. 2, mars 1985, AMSTERDAM NL, pages 323-326, XP002041837 cité dans la demande**
- **CHAPUS C. ET AL.: "Role of colipase in the interfacial adsorption of pancreatic lipase at hydrophylic interfaces" FEBS LETTERS, vol. 58, no. 1, octobre 1975, AMSTERDAM NL, pages 155-158, XP002041838 cité dans la demande**
- **MAHE-GOUHIER N. AND LEGER C.L.: "Immobilized colipase affinities for lipases B, A, C, and their terminal peptide (336-449): the lipase recognition site lysine residues are located in the C-terminal region" BIOCHIMICA ET BIOPHYSICA ACTA, vol. 962, 1988, AMSTERDAM, NL, pages 91-97, XP002041839 cité dans la demande**
- **MELIA A.T. ET AL.: "The effect of orlistat, an inhibitor of dietary fat absorbtion, on the absorbtion of vitamins A and E in healthy volunteers" JOURNAL OF CLINICAL PHARMACOLOGY, vol. 36, no. 7, juillet 1996, pages 647-653, XP002043023**
- **CHENG Q. ET AL.: "C-terminal domain of apolipoprotein C II as both activator and competitive inhibitor of lipoprotein lipase" BIOCHEMISTRY JOURNAL, vol. 269, no. 2, 15 juillet 1990, pages 403-407, XP002043024**

## Description

La présente invention est relative à des inhibiteurs spécifiques de la lipase pancréatique et à leurs applications dans le traitement et la prévention des maladies cardiovasculaires, de l'hyperlipémie et de l'obésité, ainsi qu'à leur utilisation comme réactif de diagnostic ou pour la préparation d'un médicament destiné à ménager la lipolyse de substrats triglycérides.

Les graisses alimentaires représentent une source efficace d'énergie pour l'organisme. En effet, la quantité d'énergie métaboliséè à partir des lipides est significativement plus importante que celle métabolisée à partir des carbohydrates ou des protéines. Cependant, la quasi-totalité des lipides ingérés étant assimilés par l'organisme, une surcharge alimentaire en lipides peut provoquer des troubles de santé importants : troubles cardiovasculaires, hyperlipémies et obésité.

Ces troubles sont fréquemment rencontrés dans les pays industrialisés où les populations ont souvent des régimes alimentaires trop riches en graisses saturées.

Pour lutter contre ces différentes pathologies, une hygiène alimentaire consistant à limiter l'ingestion de corps gras est nécessaire mais pas toujours suffisante. En effet, si certains corps gras sont faciles à détecter et à éliminer de l'alimentation (beurre, huiles...), d'autres, de par leur intégration dans les aliments (viandes, laitages..) le sont beaucoup moins.

Dans les cas où la mise en place d'un régime alimentaire s'avère insuffisante, des traitements pharmacologiques sont alors proposés. La plupart des traitements actuels visent surtout à lutter contre les hyperlipémies en utilisant, entre autres, des inhibiteurs de la synthèse du cholestérol.

Mais, les recherches actuelles se tournent, de plus en plus, vers des produits induisant une inhibition plus générale de l'activité lipolytique.

Dans une telle perspective, l'une des orientations qui a été particulièrement étudiée est celle de l'inhibition de la lipase pancréatique, qui est une enzyme-clé de la digestion des triglycérides alimentaires ; la digestion de ces derniers, constituants majeurs des lipides (environ 95%), amorcée dans le tractus digestif supérieur par les lipases d'origine préduodénales (lipase gastrique chez l'homme), est essentiellement réalisée dans l'intestin, sous l'action de la lipase pancréatique. Cette dernière transforme les triglycérides en acides gras libres et en 2-monoglycérides, produits d'hydrolyse plus polaires, capables de franchir la membrane de la bordure en brosse des entérocytes, après incorporation dans des micelles mixtes de sels biliaires et phospholipides.

La lipase pancréatique joue donc un rôle dans l'émergence de maladies liées à la présence d'un excès de lipides, telles que les maladies cardiovasculaires, les hyperlipémies et l'obésité, en permettant l'assimilation de la quasi-totalité des triglycérides ingérés. En outre, elle favorise l'absorption intestinale du cholestérol, dans la mesure où la solubilité du cholestérol est augmentée dans les micelles mixtes, riches en acides gras.

L'action de la lipase pancréatique comporte plusieurs étapes : adsorption de l'enzyme, dans l'intestin, sur l'interface lipidique, en présence de sels biliaires et d'une colipase, dont le rôle est d'ancrer la lipase sur l'interface (C. Chapus et al. , FEBS Letters, 1975, **58**, 1, 155-158), suivie de l'hydrolyse des liaisons esters *sn*-1,3 des triacylglycerols.

Ainsi la digestion des triglycérides fait intervenir des interactions lipase/colipase, régulées par une interface lipidique.

La lipase porcine, par exemple est une glycoprotéine comportant 449 aminoacides, dont les chaînes glycanes sont liées à l'asparagine (Asn) en position 166 ; elle comporte deux domaines séparés par la liaison $Phe^{336}-ala^{337}$ (M. Bousset-Risso et al., FEBS Letters, 1985, **182**, 2, 323-326 ). Chaque domaine porte un site de reconnaissance, à savoir : un site de reconnaissance interfaciale (domaine N-terminal), site de l'hydrolyse proprement dite et un site de reconnaissance pour son partenaire protéique (domaine C-terminal), la colipase. Le domaine N-terminal (résidus 1-335), qui porte le centre actif de l'enzyme, est séparé du domaine C-terminal (résidus 336-449), par une zone étranglée très résistante à la protéolyse. Cette organisation en deux domaines répond aux deux fonctions spécifiques énoncées ci-dessus : le domaine N-terminal est responsable de la catalyse, alors que le domaine C-terminal est impliqué dans la reconnaissance de la colipase (A. Abousalham et al., Protein Engineering, 1992, **5**, 1,105-111).

La colipase est une petite molécule (10 kDa), très réticulée du fait de la présence de 5 ponts disulfures. Elle porte trois sites de reconnaissance essentiels à sa fonction, à savoir : un site de reconnaissance interfaciale (H. van Tilbeurgh et al., Nature, 1993, **362**, 814-820), un site de reconnaissance de la lipase, (C. Chaillan et al., FEBS Letters, 1989, **257**, 2, 443-446; H. van Tilbeurgh et al., Nature, 1992, **359**, 159-162) et un site de reconnaissance micellaire (J. Hermoso et al., EMBO J., 1997, **16**, 18, 5531-5536). Ces trois sites sont topologiquement distincts.

Des études extensives réalisées depuis plusieurs années, ont permis d'approfondir les connaissances sur les relations structure/fonction du système lipase/colipase pancréatiques (C. Chaillan et al., 1989, précité).

Des recherches structurelles complémentaires concernant tant la lipase humaine (Winkler F.K. et al., Nature, 1990, **343**, 771-774) que la lipase de cheval (B. Kerfelec et al., Eur. J. Biochem., 1992, **206**, 279-287 ; Y. Boume et al., J. Mol. Biol.,1994, **238**, 709-732) ont permis de confirmer l'existence des deux domaines précités, dans des lipases de différentes origines.

La reconnaissance entre la lipase et la colipase fait intervenir en particulier des interactions hydrophobes (N. Mahé-Gouhier et al., BBA, 1988, **962**, 91-97) et électrostatiques.

Des expériences de pontage covalent entre la lipase et la colipase pancréatiques (C. Chaillan et al. 1989, précité), ainsi que la résolution de la structure tridimensionnelle à 3,1 Å du complexe lipase/colipase (H. van Tilbeurgh et al., 1992, précité) ont conduit à l'identification sur les deux molécules des zones de reconnaissance, en solution.

Pour inhiber la lipase pancréatique et obtenir une activité thérapeutique sur les hyperlipémies et l'obésité, différentes approches ont été proposées :

- l'utilisation d'inhibiteurs covalents, qui se fixent au centre actif de l'enzyme ; on peut citer, par exemple, la tétrahydrolipstatine (THL) [Brevet US 4 598 089 ; P. Hadvary et al., J. Biol. Chemistry, 1991, **266**, 4, 2021-2027 ; D. Hermier et al., FEBS Letters, 1991, **286**, 1 186-188] ; on obtient une inhibition complète de l'activité lipolytique pour des doses de 1 mol de THL/mol d'enzyme (P. Hadvary et al., 1991, précité) ou des doses de 10 à 400 mg, deux fois par jour (J. Hauptman et al., Am. J. Clin. Nutr., 1992, **55**, 309S-313S) ; une telle méthode présente un certain nombre d'inconvénients, dont le principal est le manque de spécificité : la tétrahydrolipstatine n'est en effet pas spécifique de la lipase pancréatique et inhibe d'autres lipases telles que la carboxylester lipase, la lipase gastrique et la lipase stimulée par les sels biliaires du lait humain ; et
- la modification de la nature de l'interface par adjonction de protéines amphiphiles (Gargouri Y. et al., J. Biol. Chem., 1985, **260**, 2268-2273), de détergents (Gargouri Y. et al., J. Lip. Res., 1983, **24**, 1336-1342) ou de fibres (Borel P. et al., Am. J. Clin. Nutr., 1989, **49**, 1192-1202) ; une telle méthode présente une efficacité très relative.

Ces deux approches comportent, en outre, un risque non négligeable d'effets indésirables (nausées...).

En conséquence, le Demandeur s'est donné pour but de pourvoir à un nouvel inhibiteur de la lipase pancréatique, qui réponde mieux aux besoins de la pratique que les inhibiteurs de la lipase de l'art antérieur, notamment en ce qu'il présente une réelle spécificité d'action vis-à-vis de la lipase pancréatique.

La présente invention a pour objet un peptide constitué d'un fragment C-terminal d'une lipase pancréatique, incluant le site de reconnaissance d'une colipase (dénommé ci-après peptide C-terminal), pour son utilisation comme médicament, notamment pour le traitement des hyperlipémies, des maladies cardiovasculaires et de l'obésité.

Selon un mode de réalisation avantageux de l'invention ledit peptide est un fragment C-terminal d'une lipase pancréatique, sélectionnée parmi les lipases pancréatiques humaine, porcine ou chevaline, purifiées ou recombinantes.

De manière inattendue, le peptide C-terminal de ces différentes lipases pancréatiques permet effectivement de servir de leurre et d'instaurer une compétition entre ce peptide et la lipase native, pour la colipase et ainsi de diminuer de manière significative l'action lipolytique de ladite lipase.

L'administration d'un tel peptide freine l'action de la lipase pancréatique et inhibe, de manière surprenante, au moins en partie, l'hydrolyse des triglycérides alimentaires, qui ne seront donc pas absorbés (effet inhibiteur du peptide C-terminal sur la lipolyse).

En effet, l'affinité de ce peptide C-terminal est, *in vitro* :

- en solution, de l'ordre de $10^6$ vis-à-vis de la colipase, alors que
- à l'interface lipidique, l'affinité du peptide C-terminal est de l'ordre de $2.10^8$ M.

Pour obtenir l'action recherchée, c'est-à-dire une affinité à l'interface, de l'ordre de $2.10^8$ M, ledit peptide C-terminal est, de préférence, administré aux doses de 0,5 à 10 mg/jour réparties en 1 à 3 prises, correspondant à des doses de 0,2 à 10 mg par prise.

La présente invention a également pour objet une composition pharmaceutique comprenant le peptide C-terminal d'une lipase pancréatique, incluant le site de reconnaissance d'une colipase, tel que défini ci-dessus et au moins un véhicule pharmaceutiquement acceptable.

Ladite composition pharmaceutique se présente avantageusement sous forme unitaire apte à être administrée par voie orale, sélectionnée dans le groupe consistant en capsules molles ou dures, comprimés, solutions, suspensions et émulsions.

Une telle composition pharmaceutique est, de préférence, destinée à une administration orale, sous une forme gastro-résistante.

La présente invention a également pour objet d'autres applications dudit peptide C-terminal de lipase pancréatique, par exemple en tant que réactif de diagnostic, notamment dans la mise en oeuvre d'un test immunoenzymatique de dosage de la lipase par une méthode de type compétitif ainsi que son utilisation pour la préparation d'un médicament destiné à ménager la lipolyse de substrats triglycéridiques.

Outre les dispositions qui précèdent, l'invention comprend encore d'autres dispositions, qui ressortiront de la description qui va suivre, qui se réfère à des exemples de mise en oeuvre de la présente invention, ainsi qu'aux dessins annexés dans lesquels :

- les figures 1 et 2 représentent les tracés de Scatchard obtenus à partir des spectres différentiels [(colipase-peptide C-terminal)-peptide C-terminal] en solution aqueuse pour la figure 1 et en présence d'une interface tributyrine/eau pour la figure 2;

- les figures 3 et 4 représentent l'inhibition de l'activité lipase par le peptide C-terminal de la lipase (substrat : trioléine, phospholipides, sels biliaires totaux) en l'absence (figure 3) ou en présence (figure 4) d'acide oléique.

Il doit être bien entendu, toutefois, que ces exemples sont donnés uniquement à titre d'illustration de l'objet de l'invention, dont ils ne constituent en aucune manière une limitation.

### Exemple 1 : Isolement du Peptide C-terminal de porc.

- Purification de la lipase pancréatique et de la colipase

La lipase pancréatique porcine est purifiée comme décrit dans Rovery M. et al. (Biochim. Biophys. Acta, 1978, **525**, 373-379), à partir d'une poudre pancréatique acétonique.

La purification de la colipase de porc est réalisée selon C. Chapus et al. (Eur. J. Biochem., 1982, **115**, 99-105).

Les concentrations en protéine sont déterminées à 280 nm, en utilisant un coefficient d'absorption moléculaire de $6,65.10^4$ $M^{-1}$ $cm^{-1}$, pour la lipase et de $0,4. 10^4$ $M^{-1}$ $cm^{-1}$ pour la colipase.

- Obtention, purification et analyse du peptide C-terminal de la lipase pancréatique de porc

100 mg de lipase porcine est dissous dans 20 ml de tampon bicarbonate d'ammonium 60 mM, pH 8,5, contenant de la benzamidine 1 mM.

1 mg de chymotrypsine traitée au TLCK (tosyl-lysine chlorométhyl cétone) est ajoutée et le mélange est incubé à 37°C, pendant 18 heures. Des aliquots sont prélevés à différents moments, analysés par SDS-PAGE et testés pour leur activité lipasique.

L'attaque chymotrypsique est stoppée par addition de PMSF 0,2 mM (phénylméthyl sulfonyl fluorure).

Le mélange de digestion est soumis à un tamisage moléculaire sur colonne Ultrogel AcA 54 (2,5 x 200 mm), équilibrée avec un tampon Tris-HCl 10 mM, pH 7,5, contenant 0,2 M de NaCl et 1 mM de benzamidine ; l'élution est réalisée avec le même tampon.

L'analyse en aminoacides est réalisée avec un analyseur automatique (Beckman 6300), après 24 heures d'hydrolyse des échantillons, dans des tubes scellés contenant de l'HCl tridistillé 6 M. La séquence N-terminale du peptide est obtenue par dégradation, selon la méthode d'Edman, en utilisant un séquenceur (Applied Biosystems 470A). Les phénylthiohydantoïnes obtenues sont analysées par HPLC (colonne C18, Brownlee, 5 μm, 2,1 x 220 mm). Elles sont éluées en utilisant un gradient de méthanol (10-46 %) dans un tampon acétate de sodium 5 mM, pH 4,84.

Les fragments obtenus sont soumis à une électrophorèse sur gel (PAGE-SDS), selon la technique de Laemmli U.K. (Nature, 1970, 227, 680-685). Les protéines et les fragments peptidiques sont colorés au bleu brillant de Coomassie et des tranches de gel sont décolorées à l'aide d'un mélange éthanol:acide acétique: eau (5:7,5:87,5 vol/vol).

Les peptides générés par l'attaque protéolytique sont chargés, en double, sur des fragments de gel de polyacrylamide à 18 % et séparés par électrophorèse en présence de SDS.

Après transfert sur PVDF (polyvinylidène difluorure), une piste est colorée pour localiser le peptide C-terminal et l'autre est coupée pour réaliser le séquençage de cette séquence.

L'analyse du peptide C-terminal, isolé et purifié, par SDS-PAGE ainsi que l'analyse en aminoacides, montre que ce peptide a le poids moléculaire et la composition en aminoacides attendue (voir N. Mahé-Gouhier et al., 1988, précité et M. Bousset-Risso et al., 1985, précité). Le séquençage de la partie N-terminale de la chaîne présente la séquence suivante : Ala-Arg-Trp-Arg-Tyr-Lys-Val-Ser, montrant clairement que la chymotrypsine clive la séquence au niveau de la liaison $Phe^{336}$-$Ala^{337}$, qui est à la jonction des domaines C- et N- terminaux de la lipase pancréatique.

### Exemple 2 Mise en évidence de l'interaction peptide C-terminal de la lipase porcine avec la colipase, en solution aqueuse.

L'interaction domaine C-terminal/colipase en milieu aqueux (en l'absence d'interface lipidique) a été mesurée par spectrofluorométrie.

Les spectres d'émission de fluorescence du peptide C-terminal sont enregistrés sur un spectrofluoromètre Kontron (SFM235), équipé d'une cellule thermostatée et d'un système d'agitation magnétique. Ce spectrofluoromètre est en outre connecté à un ordinateur (Apple IIe).

Toutes les expériences ont été réalisées à 25°C et à pH 7,5. La longueur d'onde d'excitation de 290 nm a été choisie parce que la colipase pancréatique de porc ne comporte pas de résidus tryptophane et les changements dans l'émission de fluorescence peuvent ainsi être attribués aux résidus tryptophane du peptide C-terminal, lorsqu'il interagit avec la colipase.

Le spectre d'émission de fluorescence du peptide C-terminal à une concentration finale de $0,26.10^{-6}$ M dans le tampon Tris-HCl 10 mm, pH 7,5, contenant du NaCl 0,1 M, est mesuré entre 300 et 400 nm. La solution mère de colipase est préparée dans le même tampon ; la concentration de colipase varie de $2,8.10^{-8}$ à $1,4.10^{-6}$ M.

L'évaluation de la constante de dissociation est déterminée en utilisant la formule de Scatchard suivante :

$$\frac{L_b}{L_f} = \frac{nP_o}{K_d} - \frac{L_b}{K_d}$$

dans laquelle $L_b$ et $L_f$ correspondent aux concentrations de colipase liée et de colipase libre à l'équilibre, $P_o$ la concentration totale en peptide C-terminal et $K_d$, la constante de dissociation.

- Résultats

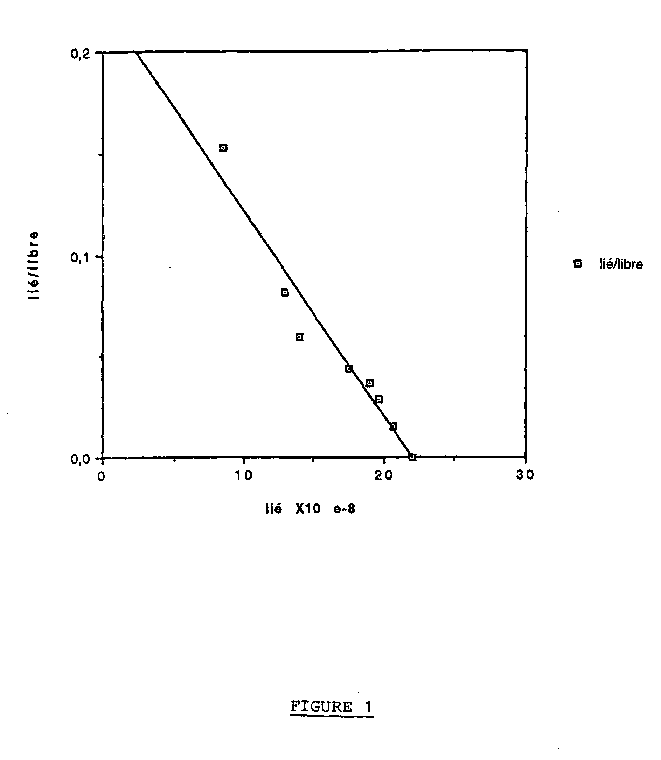

Les spectres différentiels [(colipase-peptide C-terminal)-peptide C-terminal] montrent une diminution de l'émission de fluorescence. Les tracés de Scatchard, obtenus à partir de ces spectres permettent d'évaluer la constante de dissociation colipase/peptide C-terminal à $10^{-6}$ M en solution, dans les conditions précisées ci-dessus (figure 1).

Dans cette figure 1, les abscisses représentent la quantité de peptide C-terminal lié [complexe colipase/C-terminat(TC)] et les ordonnées représentent le rapport peptide C-terminal liélpeptide C-terminal libre [complexe colipase/C-terminal(TC)/C-terminal libre(C) (TC/T)].

**Exemple 3 : Mise en évidence de l'interaction domaine C-terminal de la lipase porcine avec la colipase et action inhibitrice *in vitro* sur la lipase, à l'interface (substrat : tributyrine).**

- conditions de mesure de l'inhibition des activités lipase et colipase par le peptide C-terminal de la lipase, en présence d'une interface

L'activité lipasique est mesurée par titrimétrie, à 25°C dans un milieu constitué de tampon Tris-HCl 1 mM à pH 7,5, contenant du NaCl 0,1 M et du $CaCl_2$ 5 mM, en présence de taurodésoxycholate de sodium (NaTDC) à une concentration finale de 1 mM

L'activité colipasique est mesurée dans les mêmes conditions, mais en présence de NaTDC 4 mM.

Le volume final est de 15 ml ; le substrat utilisé est la tributyrine émulsifiée, à une concentration finale de 0,11 M.

Les activités sont mesurées en l'absence et en présence de quantités croissantes d'une solution mère de peptide C-terminal ($10^{-6}$ M).

- inhibition de l'activité lipase par le peptide C-terminal de la lipase

Le rapport molaire lipase/colipase est égal à 1 et la concentration finale de lipase et de colipase est de $0,19.10^{-9}$ M.

La concentration de peptide C-terminal varie de $0,2.10^{-9}$ à $30.10^{-9}$ M.

Le taux d'inhibition maximum de l'activité lipase enregistré dans ces conditions est de 50 % environ.

- inhibition de l'activité colipase par le peptide C-terminal de la lipase

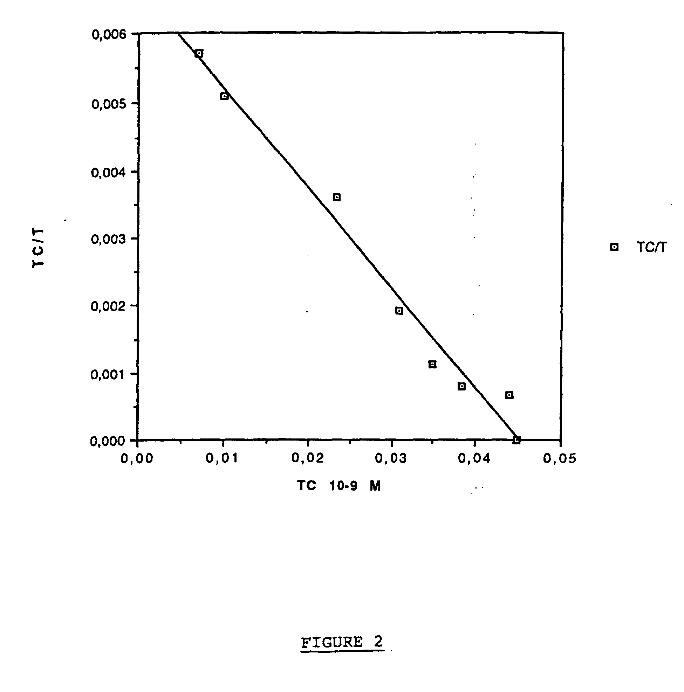

Le rapport molaire lipase/colipase est égal à 0, 75 (lipase $0,19.10^{-9}$ M ; colipase $0,25.10^{-9}$ M). La concentration en peptide C-terminal varie comme précédemment. Le taux d'inhibition maximum de l'activité colipase enregistré est également de 50 %. La constante de dissociation estimée à partir du tracé de Scatchard est de $2.10^{-8}$ M (figure 2).

Ces résultats montrent que, en solution, l'affinité de la colipase pour le peptide C-terminal est du même ordre de grandeur que l'affinité de la colipase pour la lipase et que cette affinité est augmentée d'un facteur 50 à 100 en présence d'une interface tributyrine/eau.

**Exemple 4 : Mise en évidence de l'interaction du domaine C-terminal de la lipase avec la colipase en présence d'une interface lipidique: inhibition de l'activité lipasique par le domaine C-terminal *in vitro* (substrat physiologique : trioléine émulsifiée).**

Les études cinétiques d'inhibition de la lipase par le domaine C-terminal ont été réalisées en présence d'une interface lipidique plus physiologique qu'à l'exemple 3, le substrat utilisé étant de la trioléine émulsifiée.

L'activité de la lipase est mesurée par titrimétrie à l'aide d'un pH stat, à 25°C, sur une émulsion de trioléine dans des conditions où l'activité de l'enzyme est strictement dépendante de la présence de la colipase.

Le milieu réactionnel (15 ml) contient :

- trioléine 8 mM
- taurodésoxycholate de sodium 4 mM
- Tris/HCl 1 mM, pH 7,5, $CaCl_2$ 5 mM, NaCl 0,1 M.

Les expériences de compétition sont réalisées en présence de lipase et colipase natives et du domaine C-terminal obtenu par protéolyse.

Inhibition de l'activité lipase par le domaine C-terminal :

Les concentrations finales de lipase et colipase sont de $1,7x10^{-9}$ M et $1,4x10^{-9}$M respectivement.

Le rapport molaire lipase/colipase est égal à 1,2. Pour une concentration en domaine C-terminal égale à $2x10^{-7}$ M, le taux d'inhibition maximum observé est de 50 % environ.

**Exemple 5 : Mise en évidence de l'interaction du domaine C-terminal de la lipase porcine avec la colipase et action inhibitrice *in vitro* sur la lipase, à l'interface, dans des conditions plus physiologiques**

**(substrat : trioléine, phospholipides et sels biliaires totaux).**

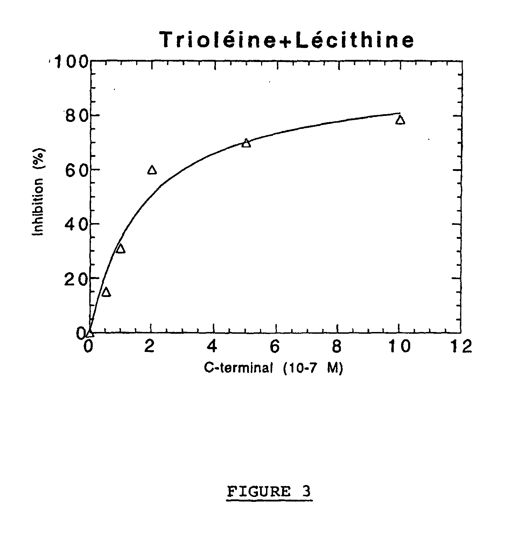

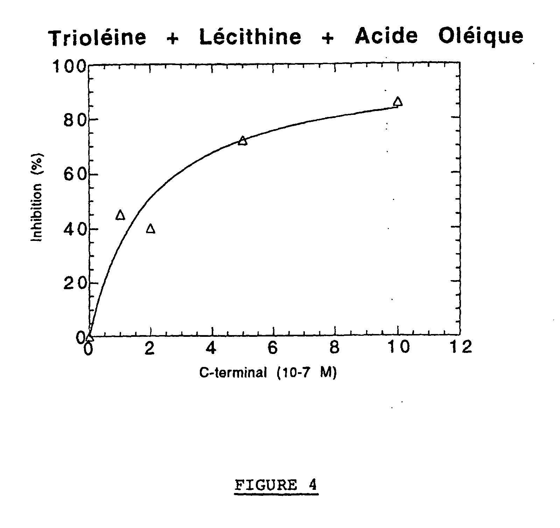

- L'inhibition de l'activité lipase par le peptide C-terminal de la lipase est mesurée comme décrit dans l'exemple 3, mais les substrats utilisés consistent en des émulsions de triglycérides à chaînes longues (trioléine 10 mM) en présence de phospholipides (rapport trioléine/lécithine = 20) et d'un mélange de sels biliaires totaux (6 mM) en l'absence (figure 3) ou en présence (figure 4) d'acide oléique (5 mM). L'acide oléique est un produit de la lipolyse. Lors de l'arrivée du bol alimentaire dans le duodénum, une partie des triglycérides alimentaires a été hydrolysée et des acides gras à chaîne longue (tels l'acide oléique) sont présents dans le duodénum. Ces acides gras libres vont se combiner à la sécrétion biliaire et se retrouver également en partie à l'interface lipidique. Ils jouent donc un rôle dans la lipolyse:

- Résultats :

Dans tous les cas, 50 % d'inhibition de la lipolyse sont obtenus en présence de $2.10^{-7}$ M de peptide C-terminal, ce qui représente une augmentation de 5 fois de l'affinité du domaine C-terminal pour la colipase en présence de l'interface lipidique, par comparaison avec les valeurs obtenues en l'absence de l'interface lipidique (figure 1).

Ainsi que cela ressort de ce qui précède, l'invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée de la présente invention.

**Revendications**

1. Peptide constitué d'un fragment C-terminal de lipase pancréatique incluant le site de reconnaissance d'une colipase, pour son utilisation comme médicament.

2. Peptide selon la revendication 1, **caractérisé en ce qu'**il correspond à un fragment d'une lipase pancréatique sélectionnée parmi les lipases pancréatiques humaine, porcine ou chevaline, purifiées ou recombinantes.

3. Peptide selon la revendication 1 ou la revendication 2, pour son utilisation dans le traitement des hyperlipémies.

4. Peptide selon la revendication 1 ou la revendication 2, pour son utilisation dans le traitement de l'obésité.

5. Peptide selon la revendication 1 ou la revendication 2, pour son utilisation dans le traitement des troubles cardiovasculaires.

6. Composition pharmaceutique comprenant un peptide selon l'une quelconque des revendications 1 à 5 et au moins un véhicule pharmaceutiquement acceptable.

7. Composition pharmaceutique selon la revendication 6, **caractérisée en ce que** la quantité dudit peptide est comprise entre 0,2 et 10 mg.

8. Composition pharmaceutique selon la revendication 6 ou la revendication 7, **caractérisée en ce qu'**elle se présente sous une forme unitaire apte à être administrée par voie orale, sélectionnée dans le groupe consistant en capsules molles ou dures, comprimés, solutions, suspensions et émulsions.

9. Composition selon la revendication 8, **caractérisée en ce que** ladite forme unitaire est gastro-résistante.

10. Utilisation d'un peptide selon la revendication 1 ou la revendication 2 comme réactif de diagnostic.

11. Utilisation d'un peptide selon la revendication 1 ou la revendication 2 pour la préparation d'un médicament destiné à ménager la lipolyse de substrats triglycéridiques.

**Patentansprüche**

1. Peptid, das aus einem C-Endgruppenfragment von Pankreaslipase besteht, das die Erkennungsstelle für eine Colipase enthält, für seine Verwendung als Medikament.

2. Peptid nach Anspruch 1, **dadurch gekennzeichnet, dass** es einem Fragment einer Pankreaslipase entspricht, die aus gereinigten oder rekombinanten Pankreaslipasen von Mensch, Schwein oder Pferd ausgewählt ist.

3. Peptid nach Anspruch 1 oder Anspruch 2, für seine Verwendung in der Behandlung von Hyperlipämien.

4. Peptid nach Anspruch 1 oder Anspruch 2, für seine Verwendung in der Behandlung von Adipositas.

5. Peptid nach Anspruch 1 oder Anspruch 2, für seine Verwendung in der Behandlung von kardiovaskulären Störungen.

**6.** Pharmazeutische Zusammensetzung, die ein Peptid nach einem der Ansprüche 1 bis 5 und mindestens einen pharmazeutisch zugelassenen Arzneistoffträger umfasst.

**7.** Pharmazeutische Zusammensetzung nach Anspruch 6, **dadurch gekennzeichnet, dass** die Menge des Peptids zwischen 0,2 und 10 mg beträgt.

**8.** Pharmazeutische Zusammensetzung nach Anspruch 6 oder Anspruch 7, **dadurch gekennzeichnet, dass** sie sich in einer Einheitsform darstellt, die sich zur Verabreichung auf oralem Weg eignet, die aus der Gruppe ausgewählt ist, die aus Weich- oder Hartkapseln, Tabletten, Lösungen, Suspensionen und Emulsionen besteht.

**9.** Zusammensetzung nach Anspruch 8, **dadurch gekennzeichnet, dass** sie magensaftresistent ist.

**10.** Verwendung eines Peptids nach Anspruch 1 oder Anspruch 2 als Diagnosereagens.

**11.** Verwendung eines Peptids nach Anspruch 1 oder Anspruch 2 zur Herstellung eines Medikaments, das dazu bestimmt ist, die Lipolyse von Triglyceridsubstraten zu hemmen.

**Claims**

**1.** Peptide consisting of a C-terminal fragment of pancreatic lipase including the recognition site for a colipase, for its use as a medicament.

**2.** Peptide according to Claim 1, **characterized in that** it corresponds to a fragment of a pancreatic lipase selected from purified or recombinant human, porcine or equine pancreatic lipases.

**3.** Peptide according to Claim 1 or Claim 2, for its use in the treatment of hyperlipaemias.

**4.** Peptide according to Claim 1 or Claim 2, for its use in the treatment of obesity.

**5.** Peptide according to Claim 1 or Claim 2, for its use in the treatment of cardiovascular disorders.

**6.** Pharmaceutical composition comprising a peptide according to any one of Claims 1 to 5 and at least one pharmaceutically acceptable vehicle.

**7.** Pharmaceutical composition according to Claim 6, **characterized in that** the quantity of the said peptide is between 0.2 and 10 mg.

**8.** Pharmaceutical composition according to Claim 6 or Claim 7, **characterized in that** it is provided in a unit form capable of being administered by the oral route, selected from the group consisting of soft or hard gelatin capsules, tablets, solutions, suspensions and emulsions.

**9.** Composition according to Claim 8, **characterized in that** the said unit form is gastroresistant.

**10.** Use of a peptide according to Claim 1 or Claim 2 as a diagnostic reagent.

**11.** Use of a peptide according to Claim 1 of Claim 2 for the preparation of a medicament intended for controlling the lipolysis of triglyceride substrates.

FIGURE 1

FIGURE 2

Trioléine+Lécithine

FIGURE 3

## Trioléine  +  Lécithine  +  Acide  Oléique

FIGURE 4